# EUROPÄISCHE PATENTANMELDUNG

(11) **EP 1 394 140 A1**
(43) Veröffentlichungstag der Anmeldung: **03.03.2004**
(21) Anmeldenummer: 03017576.4
(22) Anmeldetag: 07.08.2003
(51) Int. Cl.: C07B 53/00, C07C 67/00, C07C 69/608, C07C 69/612, C07D 333/10

(54) **Enantioselektives Reformatsky-Verfahren zur Herstellung von optisch aktiven Alkoholen, Aminen und deren Derivaten**

(30) Priorität: 14.08.2002 DE 10237275
(71) Anmelder: Consortium für elektrochemische Industrie GmbH, 81379 München (DE)
(72) Erfinder: Sorger, Klas, Dr., 81371 München (DE); Petersen, Hermann, Dr., 84489 Burghausen (DE); Stohrer, Jürgen, Dr., 82049 Pullach (DE)
(74) Vertreter: Schuderer, Michael, Dr.

(57) **Zusammenfassung**

Gegenstand der Erfindung ist ein Verfahren zur Herstellung von optisch aktiven Alkoholen, Aminen und deren Derivaten durch Umsetzung von Aldehyden oder Iminen bei einer Temperatur von kleiner 15 °C in Gegenwart eines chiralen, enantiomerenreinen Auxiliars mit einem Organozinkhalogenid, welches aus Zink und einer reaktiven Halogenverbindung erzeugt wird und anschließend Wasser, Säure oder Base oder ein Alkylierungs-, Arylierungs-, Acylierungs- oder Silylierungsreagens zugegeben wird.

## Beschreibung

Die Erfindung betrifft ein enantioselektives Reformatsky-Verfahren zur Herstellung von optisch aktiven Alkoholen, Aminen und deren Derivaten.

Die Umsetzung von reaktiven Halogenverbindungen, insbesondere α-Halogencarbonylverbindungen mit elektrophilen Substraten wie z. B. Aldehyden, Ketonen oder Iminen in Gegenwart von Zinkmetall, bekannt als Reformatsky-Reaktion, liefert wichtige Synthesebausteine für die Herstellung von pharmazeutischen Wirkstoffen, Duftstoffen und Pflanzenschutzmitteln.

Asymmetrische Reformatsky-Reaktionen zur gezielten Herstellung, von enantiomerenreinen Synthesebausteinen wie z. B. Alkoholen und Aminen gewinnen dabei immer mehr an Bedeutung.

Aus M. Guette, J. Capillon, J.-P. Guette, Tetrahedron, 1973, 29, S. 3659 ist beispielsweise eine enantioselektive Reformatsky-Synthese von (S)-(-)-3-Hydroxy-3-phenylpropionsäureestern unter Verwendung von Benzaldehyd als Substrat in Gegenwart des chiralen Liganden (-)-Spartein in den wenig koordinierenden Lösungsmitteln Benzol und Dimethoxymethan bekannt.
Das Verfahren ermöglicht einerseits hohe Enantioselektivitäten von bis zu 95±3 % ee, andererseits konnten nur sehr geringe chemische Ausbeuten von maximal 38±7 % der Theorie erzielt werden.

Pedrosa et al. (J. M. Andrés, Y. Martín, R. Pedrosa, A. Perez-Encabo, Tetrahedron, 1997, 53, S. 3787) beschreiben eine enantioselektive Reformatsky-Reaktion zur Herstellung optisch aktiver β-Hydroxyester mit Aminoalkoholen als chirale Auxiliare in Tetrahydrofuran.
Jedoch werden bei hohen chemischen Ausbeuten bis zu 90 % nur mäßige Enantioselektivitäten von 22 bis 62 % ee erreicht. Das Verfahren basiert zudem auf dem Einsatz hoher Überschüsse der Organozinkverbindung (Reformatsky-Reagenz) und ist somit aus wirtschaftlicher und großtechnischer Sicht uninteressant.

Seebach et al. (D. Seebach, W. Langer, Helv. Chim. Acta 1979, 62, S. 1701) beschreiben eine enantioselektive Reformatsky-Reaktion mit chiralen Diaminen als Cosolventien mit guten chemischen Ausbeuten von bis zu 95 %, aber nur niedrigen Enantiomerenüberschüssen von maximal 24.5 % ee.

Ojida et al. (A. Ojida, T. Yamano, N. Taya, A. Tasaka, Org. Lett. 2002, 4, S. 3051) beschreiben ein enantioselektives Reformatsky-Verfahren unter Einsatz von Cinchona-Aminoalkoholen als chirale Liganden und Verwendung von Ketonen als Substrate. Hohe Enantioselektivitäten (bis 97 % ee) konnten nur dann erzielt werden, wenn ein sp²-Stickstoff-Atom in Nachbarstellung zur Carbonylgruppe des Keton-Substrats eine Chelation des Zinks ermöglicht. Andere Keton-Substrate und Aldehyde als elektrophile Substrate liefern nur mäßige optische Ausbeuten von 15-70 % ee. Zudem erfordert das Verfahren einen großen Überschuss an Reformatsky-Reagenz und teurem Pyridin als basisches Hilfsmittel, was das Verfahren aus wirtschaftlicher und großtechnischer Sicht uninteressant macht.
Ukaji et al. (Y. Ukaji, S. Takenaka, Y. Horita, K. Inomata, Chem. Lett. 2001, S. 254) beschreiben ein enantioselektives Reformatsky-Verfahren zur Herstellung optisch aktiver β-Aminosäureester unter Verwendung von Diisopropyltartrat als chirales Auxiliar. Auch wenn Enantioselektivitäten von bis zu 98 % ee und Ausbeuten von bis zu 80 % erzielt werden konnten, macht das Verfahren den Einsatz hoher Überschüsse sowohl der Organozinkverbindung (Diethylzink) als auch von sehr teurem Iodessigester erforderlich. Aus wirtschaftlicher und großtechnischer Sicht ist dieses Verfahren deshalb zur Herstellung optisch aktiver β-Aminosäureester ungeeignet.

Keines der bekannten Verfahren liefert bei wirtschaftlichem Einsatz verwendeter Reagenzien insbesondere von Organo-Zinkverbindung sowohl hohe chemische wie auch hohe optische Ausbeuten. Die aus dem Stand der Technik bekannten Verfahren sind somit insbesondere für eine großtechnische und wirtschaftliche Synthese von optisch aktiven Alkoholen und Aminen ungeeignet.

Es bestand daher die Aufgabe ein wirtschaftliches, universell und großtechnisch durchführbares, enantioselektives Reformatsky-Verfahren bereitzustellen, dass im Rahmen einer enantioselektiven Synthese zu hoch enantiomerenangereicherten Alkoholen und Aminen bzw. deren Derivaten in hohen Ausbeuten führt und die aus dem Stand der Technik bekannten Probleme löst.

Es wurde überraschend gefunden, dass die Herstellung von optisch aktiven Alkoholen und Aminen und deren Derivaten in Form einer enantioselektiven Reformatsky-Reaktion unter Verwendung von chiralen, enantiomerenreinen Auxiliaren mit mindestens zwei Stickstoff-Donatoren und mindestens einer cyclischen Gruppe (N-Auxiliar) besonders vorteilhaft und wirtschaftlich und mit einer Vielzahl von Substraten durchgeführt werden kann.

Gegenstand der Erfindung ist ein Verfahren zur Herstellung von Verbindungen der allgemeinen Formel (1a) oder (1b) 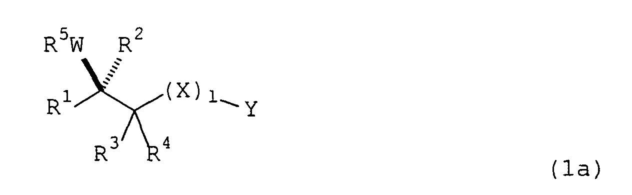 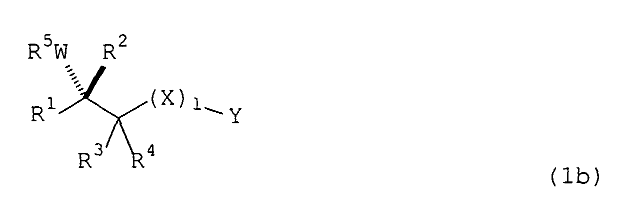 **wobei**
**R**^{**1**} und **R**^{**2**} unabhängig voneinander Wasserstoff oder einen gegebenenfalls halogen- oder cyanosubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S- oder -N**R**^{**x**}- ersetzt sein können und in dem eine oder mehrere Methineinheiten durch Gruppen -N= oder -P= ersetzt sein können,
**R**^{**3**} und **R**^{**4**} unabhängig voneinander Wasserstoff, Halogen oder einen gegebenenfalls halogensubstituierten oder cyanosubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S- oder -N**R**^{**x**}- ersetzt sein können und in dem eine oder mehrere Methineinheiten durch Gruppen -N= oder -P= ersetzt sein können,
**R**^{**x**} Wasserstoff oder einen gegebenenfalls halogensubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S-, -NHoder -N-C₁-C₂₀-Alkyl ersetzt sein können und in dem eine oder mehrere Methineinheiten durch Gruppen -N= oder -P= ersetzt sein können, bedeuten und
- **X**: ausgewählt wird aus 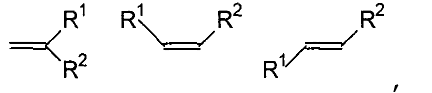
- **l**: für eine ganze Zahl im Wert von 0 oder 1 steht,
- **W**: O oder NR⁶,
**R**^{**5**} Wasserstoff oder eine Alkyl-, Aryl-, Acyl- oder Silylgruppe und
**R**^{**6**} Wasserstoff, Si(R¹)₃ oder einen gegebenenfalls halogenoder cyanosubstituierten C₁-C₃₀-Kohlenwasserstoffrest bedeuten kann, in dem eine oder mehrere, einander nicht benachbarte Methyleneinheiten durch Gruppen -O-, -CO-, -COO-, -OCO-, oder -OCOO-, -S-, -SO-, -SO₂-, -P(R¹)-, -P(OR¹)-, -PO(R¹)-, -PO(OR¹)-, oder -NR^{x}- ersetzt sein können und in dem eine oder mehrere Methineinheiten durch Gruppen -N= oder -P= ersetzt sein können,
**Y** für CN, (C=O)-Z, (SO₂)-Z, (P=O)(-Z)₂ oder einen aromatischen Rest steht, wobei eine oder mehrere Methineinheiten im Ring durch Gruppen -N= oder -P= ersetzt sein können und der Ring die Heteroatome -O-, -S- oder -NH- tragen kann, wobei der aromatische Ring gegebenenfalls halogen- oder cyanosubstituiert oder durch C₁-C₃₀-Kohlenwasserstoffreste, in denen eine oder mehrere, einander nicht benachbarte Methyleneinheiten durch Gruppen -O-, -CO-, -COO-, -OCO-, oder-OCOO-, -S- oder -NR^{x}- ersetzt sein können, substituiert ist und
**Z** für einen gegebenenfalls halogensubstituierten C₁-C₃₀-Kohlenwasserstoffrest steht, in dem eine oder mehrere, einander nicht benachbarte Methyleneinheiten durch Gruppen -O-, -CO-, -COO-, -OCO-, oder -OCOO-, -S-, oder -NR^{x}- ersetzt sein können und in dem eine oder mehrere Methineinheiten durch Gruppen -N= oder -P= ersetzt sein können, oder für OH, OR¹, OSi(R¹)₃, NHR¹ oder NR¹R² steht
**dadurch gekennzeichnet, dass**
Aldehyde oder Imine der allgemeinen Formel (2)

R¹R²C=W (2)

bei einer Temperatur von kleiner 15 °C
in Gegenwart eines chiralen, enantiomerenreinen Auxiliars mit mindestens zwei Stickstoff-Donatoren und mindestens einer cyclischen Gruppe mit der Maßgabe, dass vorhandene Protonen einen pKₐ größer 18 haben
mit einem Organozinkhalogenid umgesetzt wird, welches aus
Zink und
einer reaktiven Halogenverbindung der allgemeinen Formel (3)

Hal-R³R⁴C- (X)₁-Y (3)

wobei
**Hal** Chlor, Brom oder Iod bedeutet, erzeugt wird,
und anschließend Wasser, Säure oder Base oder ein Alkylierungs-, Arylierungs-, Acylierungs- oder Silylierungsreagens zugegeben wird.

Der synthetisch besonders wertvolle Schritt des erfindungsgemäßen Verfahrens ist der effiziente Aufbau eines chiralen Zentrums mit hohen Ausbeuten im Zuge einer Reformatsky-Reaktion mit hohen Enantiomerenüberschüssen in β-Position durch Verwendung eines chiralen enantiomerenreinen Auxiliars mit mindestens zwei Stickstoff-Donatoren und mindestens einer cyclischen Gruppe mit der Maßgabe, dass vorhandene Protonen einen pKa größer 18 haben (N-Auxiliar).

Die als chirale N-Auxiliare im erfindungsgemäßen Verfahren eingesetzten chiralen, enantiomerenreinen Verbindungen mit mindestens zwei Stickstoff-Donatoren dürfen angesichts der Reaktionsbedingungen während der Reformatsky-Reaktion und hierbei insbesondere wegen des Zusatzes von Zink keine aciden Protonen, insbesondere solche mit einem pKa kleiner oder gleich 18, zum Beispiel in Form freier Amidfunktionen, wie NH-C=O oder NH-SO₂ oder Hydroxyfunktionen besitzen. Dadurch ist das chirale Auxiliar unter den Reaktionsbedingungen der Reformatsky-Reaktion insbesondere gegenüber dem Reformatsky-Reagens inert und stabil. Folglich kann somit insbesondere auf die im Stand der Technik oftmals eingesetzten großen Überschüsse an Reformatsky-Reagenz verzichtet werden.

Besonders bevorzugte N-Auxiliare enthalten keine aciden Protonen mit einem pKₐ kleiner oder gleich 20.

Die Stickstoff-Donatoren des N-Auxiliars koordinieren an die Zinkatome und komplexieren das Reformatsky-Reagenz (Organozinkhalogenid) - selbst in Gegenwart polarer und/oder koordinierender Lösungsmittel. Das resultierende chirale Reformatsky-Reagens differenziert die re- und si-Seite des Aldehyd- oder Iminsubstrats, was zur Bildung enantiomerenangereicherter Produkte führt. Durch die Gegenwart mindestens einer cyclischen Gruppe (Cyclus) im N-Auxiliar wird die Flexibilität des Übergangszustandes bei Annäherung des chiralen Reformatsky-Reagenzes an die re- oder si-Seite des Aldehyd- oder Iminsubstrats eingeschränkt und damit die Selektivität der Reaktion erhöht, was zu hohen optischen Ausbeuten (Enantioselektivitäten) und damit hoch enantiomerenangereicherten Produkten führt.

So erreicht man beispielsweise mit dem von Seebach et al. beschriebenen Verfahren (D. Seebach, W. Langer, Helv. Chim. Acta 1979, 62, S. 1701) bei Einsatz des offenkettigen, keine cyclischen Gruppen enthaltenden Diamins (+)-1,4-Dimethylamino-2,3-dimethoxybutan als chirales Auxiliar nur niedrige Enantiomerenüberschüsse von maximal 24.5 % ee.

Üblicherweise sind die Stickstoff-Donatoren der im erfindungsgemäßen Verfahren verwendeten N-Auxiliare N-Heteroaromaten, Amin-, Imin- oder Enamin-Gruppen.

Bevorzugt sind N-Auxiliare mit mindestens zwei sekundären und/oder tertiären Amin-Gruppen enthaltend mindestens einen Cyclus.

Insbesondere sind chirale, enantiomerenreine Diaminverbindungen enthaltend mindestens einen Cyclus als Auxiliare bevorzugt, ganz besonders bevorzugt sind Diaminverbindungen mit sekundären und/oder tertiären Amin-Gruppen enthaltend mindestens eine cyclische Gruppe.

Ganz besonders bevorzugte N-Auxiliare sind Diaminverbindung mit sekundären und/oder tertiären Amin-Gruppen, in der mindestens eine Amin-Gruppe Bestandteil eines Cyclus ist.
Eine besonders bevorzugte Ausführungsform des Amino-Auxiliars ist (-)-Spartein.

Durch Verwendung von (-)-Spartein lassen sich generell hohe Enantiomerenüberschüsse der optisch aktiven Alkohole und Amine und deren Derivate der allgemeinen Formel (1a) oder (1b) erzielen. Abhängig von der Struktur des Substrats und des Reformatsky-Reagenz, der Anwesenheit weiterer funktioneller Gruppen, des verwendeten Lösungsmittels, der Temperatur und gegebenenfalls weiterer Reaktionsparameter kann dabei entweder das R- oder das S-Derivat des optisch aktiven Produkts hergestellt werden, wobei bezogen auf ein bestimmtes Substrat gezielt eine Händigkeit (die R- oder S-Form) mit einem hohem Enantiomerenüberschuss erhalten wird.

Die Vorteile des erfindungsgemäßen Verfahrens liegen darin, dass die Synthese von optisch aktiven Alkoholen, Aminen und deren Derivaten insbesondere bei der Durchführung im technischen Maßstab besonders wirtschaftlich und effizient durchführbar ist, da die Produkte in hohen Ausbeuten bei gleichzeitig hohen Reinheiten und hohen optischen Reinheiten (Enatiomerenüberschüssen) enantioselektiv in Bezug auf die 3-Oxy- oder 3-Amino-Position in den Produkten der allgemeinen Formel (1a) oder (1b) hergestellt werden können.

Zudem erfolgt die Herstellung der optisch aktiven Alkohole, Amine und deren Derivate bei Einsatz sehr geringer Überschüsse des Organozinkhalogenids (Reformatsky-Reagenz). Das Verfahren ist somit aus großtechnischer Sicht besonders wirtschaftlich.

Darüber hinaus lassen sich nach dem erfindungsgemäßen Verfahren die Produkte in sehr kurzen Reaktionszeiten und sehr guten Raum-Zeit-Ausbeuten kostengünstig gewinnen.

Ferner lässt sich das eingesetzte chirale enatiomerenreine N-Auxiliar sehr einfach bei Aufarbeitung des Reaktionsgemischs und Isolierung der Produkte in hohen Ausbeuten zurückgewinnen und wiederverwenden, was das erfindungsgemäße Verfahren insbesondere bei der Durchführung im technischen Maßstab besonders kostengünstig und wirtschaftlich macht.

Im Laufe der Umsetzung kommt es nach dem Zusatz von Wasser, Säure oder Base oder eines Alkylierungs-, Arylierungs-, Acylierungs oder Silylierungsreagens zur Abscheidung eines zinkhaltigen Feststoffes enthaltend das N-Auxiliar.

Nach Abtrennung des Niederschlags lässt sich aus dem zinkhaltigen Feststoff das N-Auxiliar durch Erwärmen einer Suspension des Feststoffes in einem Lösungsmittel in Gegenwart einer Hydroxid- oder Oxid-Base zurückgewinnen. Das N-Auxiliar wird durch diese Vorgehensweise unter Bildung schwerlöslicher basischer Zinksalze freigesetzt. Nach Filtration wird das N-Auxiliar durch Entfernung des Lösungsmittels zurückgewonnen und kann vorteilhafter Weise erneut in den Prozess zurückgeschleust werden.

So lässt sich beispielsweise (-)-Spartein bei Aufarbeitung des Reaktionsgemischs und Isolierung der Produkte in sehr hoher Ausbeute von > 90 % zurückgewinnen und wiederverwenden.

Die C₁-C₃₀-Kohlenwasserstoffreste für **R**^{**1**}**, R**^{**2**}**, R**^{**3**} **und R**^{**4**} sind vorzugsweise lineare, verzweigte oder cyclische C₁-C₂₀-Alkyl-, C₂-C₂₀-Alkenyl-, C₅-C₂₀-Acetalalkenyl-, C₃-C₂₀-Alkoxycarbonylalkylreste, die substituiert sein können durch F, Cl, Br, J, CN und C₁-C₁₀-Alkoxyreste sowie C₁-C₁₀-Alkylaminoreste und in denen Methyleneinheiten durch -O-, -S-, -N**R**^{**x**}- ersetzt sein können, Aryl-, Aralkyl-, Alkaryl-, Aralkenyl-, Alkenylarylreste, in denen eine oder mehrere Methineinheiten durch Gruppen -N= oder -P=, und in denen Methyleneinheiten durch -O-, -S- oder -N**R**^{**x**}- ersetzt sein können und die substituiert sein können durch F, Cl, Br, J, CN, C₁-C₁₀-Alkoxyreste, C₁-C₁₀-Alkylaminoreste und C₁-C₁₀-Alkylreste und die im Ring die Heteroatome -O-, -S- oder -N**R**^{**x**}- tragen können.

Besonders bevorzugte Reste **R**^{**1**} und **R**^{**2**} sind Wasserstoff, Methyl, Ethyl, Propyl, Isopropyl, Butyl, sec.-Butyl, *tert*.-Butyl, Vinyl, Phenyl; Amino-, N,N-Dimethylamino-, N-Acetylamino-, N-Acetyl-N-methylamino-, N-Benzyloxycarbonylamino-, *tert.*-Butyloxycarbonylamino-, Nitro-, Methyl-, Ethyl-, Propyl-, Isopropyl-, Butyl-, sec.-Butyl-, *tert*.-Butyl-, Methoxy-, Ethoxy-, Acetoxy-, Benzyloxy-, Fluor-, Chlor-, Brom-, Jod-, Cyanophenyl; Benzyl, Pyridyl, Piperidinyl, Thiophen, Furan, Pyrrol, insbesondere Wasserstoff, Methyl, Ethyl, Phenyl und Thiophen.

Besonders bevorzugte Reste **R**^{**3**} und **R**^{**4**} sind Wasserstoff, Fluor, Chlor, Brom oder Jod, Methyl, Ethyl, Propyl, Butyl, *tert*.-Butyl, Vinyl, Phenyl; Amino-, N,N-Dimethylamino-, N-Acetylamino-, N-Acetyl-N-methylamino-, N-Benzyloxycarbonylamino-, *tert*.-Butyloxycarbonylamino-, Nitro-, Methyl-, Ethyl-, Propyl-, Isopropyl-, Butyl-, sec.-Butyl-, *tert*.-Butyl-, Methoxy-, Ethoxy-, Acetoxy-, Benzyloxy-, Fluor-, Chlor-, Brom-, Jod-, Cyanophenyl; Benzyl, Pyridyl, Piperidinyl, Thiophen, Furan und Pyrrol, insbesondere Wasserstoff, Fluor, Chlor, Methyl und Phenyl.

Bevorzugte Reste **R**^{**5**} sind Wasserstoff, Si(R¹)₃, wobei hier R¹ insbesondere aus den bevorzugten Ausführungsformen von R¹ ausgewählt wird oder lineare, verzweigte oder cyclische C₁-C₂₀-Alkyl-, C₂-C₂₀-Alkenyl-, C₅-C₂₀-Acetalalkenyl-, C₃-C₂₀-Alkoxycarbonylalkylreste, die substituiert sein können durch F, Cl, Br, J, CN und C₁-C₁₀-Alkoxyreste sowie C₁-C₁₀-Alkylaminoreste und in denen Methyleneinheiten durch -O-, -S-, -N**R**^{**x**}- ersetzt sein können, Aryl-, Aralkyl-, Alkaryl-, Aralkenyl-, Alkenylarylreste, in denen eine oder mehrere Methineinheiten durch Gruppen -N= oder -P=, und in denen Methyleneinheiten durch -O-, -S-, -N**R**^{**x**}- ersetzt sein können und die substituiert sein können durch F, Cl, Br, J, CN, C₁-C₁₀-Alkoxyreste, C₁-C₁₀-Alkylaminoreste und C₁-C₁₀-Alkylreste und die im Ring die Heteroatome -O-, -S- oder -N**R**^{**x**}- tragen können.

Besonders bevorzugte Reste für **R**^{**5**} sind Wasserstoff, Acetyl, Propionyl, Benzoyl, Methyl, Ethyl, Propyl, Butyl, Phenyl, Benzyl, Naphtyl, Trimethylsilyl, Triethylsilyl, Tripropylsilyl, Tributylsilyl oder Butyldimethylsilyl, insbesondere Wasserstoff, Acetyl, Methyl, Naphtyl oder Trimethylsilyl.

Bevorzugte Si(R¹)₃-Reste für **R**^{**6**} sind solche, in denen R¹ aus den bevorzugten Ausführungsformen von R¹ ausgewählt wird.

Die C₁-C₃₀-Kohlenwasserstoffreste für **R**^{**6**} sind bevorzugt lineare, verzweigte oder cyclische C₁-C₂₀-Alkyl-, C₂-C₂₀-Alkenyl-, C₅-C₂₀-Acetalalkenyl-, C₃-C₂₀-Alkoxycarbonylalkylreste, die substituiert sein können durch F, Cl, Br, J, CN, Aryl-, Aralkyl-, Alkaryl-, Aralkenyl-, Alkenylarylreste, in denen eine oder mehrere Methineinheiten durch Gruppen -N= oder -P=, und Methyleneinheiten durch -O-, -S-, -NH- ersetzt sein können.

Besonders bevorzugte Reste **R**^{**6**} sind Wasserstoff, Acetyl, Benzyloxycarbonyl, *tert*.-Butyloxycarbonyl, Diphenylphosphinoyl, Phenylsulfinyl, Tolylsulfinyl, Methyl, Ethyl, Propyl, Butyl, Phenyl, Benzyl, Naphtyl, Trimethylsilyl, Triethylsiyl, Tripropylsilyl, Tributylsilyl oder Butyldimethylsilyl, insbesondere Wasserstoff, Acetyl, Methyl, Ethyl, Benzyloxycarbonyl, tert.-Butyloxycarbonyl, Diphenylphosphinoyl, Tolylsulfinyl oder Trimethylsilyl.

Bevorzugte Reste für **R**^{**x**} sind lineare, verzweigte oder cyclische C₁-C₂₀-Alkyl-, C₂-C₂₀-Alkenyl-, C₅-C₂₀-Acetalalkenyl-, C₃-C₂₀-Alkoxycarbonylalkylreste, die substituiert sein können durch F, Cl, Br, J, CN und C₁-C₁₀-Alkoxyreste sowie C₁-C₁₀-Alkylaminoreste und in denen Methyleneinheiten durch -O-, -S-, -NH- ersetzt sein können, Aryl-, Aralkyl-, Alkaryl-, Aralkenyl-, Alkenylarylreste, in denen eine oder mehrere Methineinheiten durch Gruppen -N= oder -P=, und in denen Methyleneinheiten durch -O-, -S-, -NH- ersetzt sein können und die substituiert sein können durch F, Cl, Br, J, CN, C₁-C₁₀-Alkoxyreste und C₁-C₁₀-Alkylaminoreste und durch C₁-C₁₀-Alkylreste und die im Ring die Heteroatome -O-, -S- oder -NH- tragen können.

Besonders bevorzugte Reste für **R**^{**x**} sind Wasserstoff, Methyl, Ethyl, Propyl, Butyl, tert.-Butyl, Phenyl und Benzyl, insbesondere Wasserstoff und Methyl.

Als reaktive Halogenverbindungen der allgemeinen Formel (3) werden bevorzugt reaktive α-Halogenessigsäurederivate eingesetzt.

Bevorzugt sind reaktive α-Halogenessigsäurederivate der allgemeinen Formel (3), bei der **Y** die Bedeutung CN oder (C=O)-Z hat und **Z** OR¹ und OSi(R¹)₃ bedeuten soll und R¹ insbesondere aus den oben beschriebenen bevorzugten Ausführungsformen ausgewählt wird.

Ganz besonders bevorzugte α-Halogenessigsäurederivate der allgemeinen Formel (3) sind α-Halogenessigsäurenitril, α-Halogenessigsäuremethylester, α-Halogenessigsäureethylester und α-Halogenessigsäuretrimethylsilylester.

Als reaktive Halogenverbindungen der allgemeinen Formel (3) werden insbesondere Chlor- oder Bromverbindungen, wobei **Hal** für Brom oder Chlor steht, ganz besonders bevorzugt Bromverbindungen, wobei **Hal** für Brom steht, eingesetzt.

Besonders bevorzugte Ausführungsformen für reaktive Halogenverbindungen sind α-Bromessigsäureester.

Mögliche Ausführungsformen für reaktive Bromverbindungen sind auch Reaktionsprodukte von α-Halogenessigsäurederivaten mit Silanen, wie beispielsweise Verbindungen des Typs [Br-CH₂(C=O)-O]₂Si(R¹)₂ oder [Br-CH₂(C=O)-OSi(R¹)₂-CH₂]₂, wobei R¹ die oben genannte Bedeutung haben soll, insbesondere die oben beschriebenen bevorzugten Ausführungsformen. Aus diesen werden dann durch Umsetzung mit Zink die Reformatsky-Reganzien erhalten.

In einer möglichen Ausführungsform des erfindungsgemäßen Verfahrens wird im ersten Reaktionsschritt unter Erhalt des chiralen Reformatsky-Reagenzes zuerst Zink in einem Lösungsmittel oder Lösungsmittelgemisch vorgelegt und dann eine Mischung, gegebenenfalls gelöst in einem Lösungsmittel, der reaktiven Halogenverbindung und dem N-Auxiliar zugeben.

Bevorzugt wird zuerst Zink und das N-Auxiliar in einem Lösungsmittel oder Lösungsmittelgemisch vorgelegt und anschließend die reaktive Halogenverbindung, gegebenenfalls gelöst in einem Lösungsmittel, unter Bildung eines Organozinkhalogenids der allgemeinen Formel (3), wobei Hal hier Halogenozink bedeuten soll (Organozinkhalogenid), zugegeben.

Besonders bevorzugt wird zuerst Zink in einem Lösungsmittel oder Lösungsmittelgemisch vorgelegt und anschließend die reaktive Halogenverbindung, gegebenenfalls gelöst in einem Lösungsmittel, unter Bildung eines Organozinkhalogenids zugegeben. Zu dieser Mischung wird dann das N-Auxiliar zugeben, gegebenenfalls gelöst in einem Lösungsmittel.

Die Umsetzung wird in Gegenwart eines unter den Reaktionsbedingungen inerten Lösungsmittels durchgeführt, das vor oder nach Zugabe von Zink oder dem chiralen Auxiliar oder anderer Reagenzien oder Zusatzstoffe oder gegebenenfalls zusammen mit Zink oder dem chiralen Auxiliar oder anderer Reagenzien oder Zusatzstoffe zugesetzt wird.

Als Lösungsmittel können für das erfindungsgemäße Verfahren alle für die Reformatsky-Reaktion geeigneten inerten Lösungsmittel eingesetzt werden. Eine zusammenfassende Darstellung geeigneter Lösungsmittel ist beispielsweise in A. Fürstner, Synthesis 1989, S. 571 enthalten.

Geeignete Lösungsmittel sind Diethyl-, Dipropyl-, Dibutylether, Diisopropylether, Methyl-tert.-butylether, Dimethoxymethan, Diethoxymethan, Dimethoxyethan, Diethoxyethan, Diethylenglykoldimethylether, Diethylenglykol-diethylether, Diethylenglykoldipropylether, Diethylenglykol-dibutylether, Triethylenglykoldimethylether, Triethylenglykol-diethylether, Triethylenglykol-dibutylether, Methylenchlorid sowie Gemische der vorstehend genannten Verbindungen und Gemische der vorstehend genannten Verbindungen mit den Lösungsmitteln Benzol, Toluol, Ethylbenzol, Propylbenzol, Isopropylbenzol, Butylbenzol, Xylol, Xylol-Isomerengemisch, Trimethylbenzol, Pentan, Hexan, Octan, Isooctan, Nonan, Nonan-Fraktion, Cyclohexan, Cycloheptan, Cyclooctan, Dimethylcyclohexan, Ethylcyclohexan, Propylcyclohexan, Butylcyclohexan, Petroleumbenzin und Paraffin.

In einer bevorzugten Ausführungsform werden an das Zink koordinierende oder das Zink chelatisierende Lösungsmittel oder Cosolvenzien verwendet.

Bevorzugte Lösungsmittel für die Durchführung der erfindungsgemäßen enantioselektiven Reformatsky-Reaktion sind solche ausgewählt aus der Gruppe der cyclischen Ether, Di- oder Oligoether mit mindestens einer Ethylen-Brücke (Glyme-Verbindungen), Carbonsäureester, Amine, Säureamide, polar aprotische Lösungsmittel oder halogenierte Kohlenwasserstoffe bzw. deren Gemische.

Die vorgenannten Lösungsmittel können als einziges Lösungsmittel, als Zusatz zur Reaktionsmischung oder in Mischung mit einem oder mehreren für Reformatsky-Reaktionen geeigneten inerten Lösungsmitteln oder Lösungsmittelgemischen als Cosolvenzien im erfindungsgemäßen Verfahren eingesetzt werden.

Mit Lösungsmitteln ausgewählt aus der Gruppe der cyclischen Ether, Di- oder Oligoether mit mindestens einer Ethylen-Brücke (Glyme-Verbindungen), Carbonsäureester, Amine, Säureamide, polar aprotische Lösungsmittel oder halogenierte Kohlenwasserstoffe oder Lösungsmittelgemischen enthaltend eines oder mehrerer dieser Lösungsmittel lassen sich sehr hohe Ausbeuten von bis zu > 95 % und gleichzeitig hohe Enantioselektivitäten von bis zu 95 % erreichen, bei gleichzeitig kurzen Reaktionszeiten und folglich hohen Raum-Zeit-Ausbeuten.

Besonders bevorzugte Lösungsmittel aus der Gruppe der Ether, Di- und Oligoether mit mindestens einer Ethylen-Brücke (Glyme-Verbindungen) sind Tetrahydrofuran, 2-Methyltetrahydrofuran, 2,5-Dimethyltetrahydrofuran, 2,5-Dimethoxytetrahydrofuran oder 1,4-Dioxan, Dimethoxyethan, Diethoxyethan, Diethylenglykoldimethylether, Diethylenglykol-diethylether, Diethylenglykoldipropylether, Diethylenglykol-dibutylether, Triethylenglykoldimethylether, Triethylenglykol-diethylether oder Triethylenglykol-dibutylether oder deren Gemische.

Insbesondere bevorzugt werden dabei Tetrahydrofuran, 2-Methyltetrahydrofuran, 2,5-Dimethyltetrahydrofuran, 2,5-Dimethoxytetrahydrofuran oder 1,4-Dioxan odere deren Gemische eingesetzt, wobei Tetrahydrofuran oder 1,4-Dioxan ganz besonders bevorzugt verwendet werden.

Besonders bevorzugte Lösungsmittel aus der Gruppe der Carbonsäureester, Amine, Säureamide, Ketone, polar aprotischen Lösungsmittel oder halogenierten Kohlenwasserstoffe sind Methyl-, Ethyl-, n-Propyl-, i-Propyl-, n-Butyl-, i-Butyl-, t-Butyl-, n-Hexyl-, n-Pentyl-, i-Pentylacetat, -propionat und -butyrat, 2-Essigsäure-2-ethoxyethylester, Pyridin, N-Acetylpyrrolidin, N-Acetylpyrrol, N-Acetylsuccinimid, Tetramethylharnstoff, N,N'-Dimethylethylenharnstoff, Tetramethylguanidin, Aceton, Methylethylketon, Diethylketon, Isopropylmethylketon, Isopropylethylketon, Acetonitril, Propionitril, Butyronitril, Dimethylformamid, Diethylformamid, Dimethylacetamid, Diethylacetamid, 1-Methyl-2-pyrrolidon, Dimethylsulfoxid oder Methylenchlorid oder deren Gemische. Ganz besonders bevorzugte Lösungsmittel für das erfindungsgemäße Verfahren sind Dimethylformamid, 1-Methyl-2-pyrrolidon oder Tetrahydrofuran oder deren Mischungen , insbesondere Tetrahydrofuran oder Mischungen enthaltend Tetrahydrofuran oder andere polare Lösungsmittel.

Die hergestellten Organozinkhalogenide der allgemeinen Formel (3), wobei Hal hier Halogenozink bedeuten soll (Organozinkhalogenide), können, gegebenenfalls auch nach Zugabe des chiralen N-Auxiliars, zunächst stabil gelagert und gegebenenfalls zu einem späteren Zeitpunkt zur Reaktion gebracht werden. Sie stellen wertvolle, für die enantioselektive Reformatsky-Reaktion hervorragend geeignete Reagenzien dar.

Während der Zugabe der reaktiven Halogenverbindung der allgemeinen Formel (3) und gegebenenfalls dem chiralen N-Auxiliar hält man vorzugsweise die Temperatur der exothermen Reaktion - gegebenenfalls durch Kühlung - auf einem vorgegebenen Wert. Dabei kann die obere Temperaturgrenze durch den Siedepunkt des eingesetzten Lösungsmittels, wie beispielsweise Tetrahydrofuran (Kp.: 66 °C) oder Ethylacetat (Kp.: 78 °C) begrenzt sein. Bei höhersiedenden Lösungsmitteln, wie beispielsweise 1,4-Dioxan (Kp.: 100-102 °C) wird die Temperatur der Reaktion vorzugsweise durch Kühlung kontrolliert.

Die Herstellung der Organozinkhalogenide wird vorzugsweise bei Temperaturen von -20 bis +150 °C, besonders bevorzugt bei 0 bis 100 °C, insbesondere bei 25 bis 60 °C, gegebenenfalls unter Refluxion und gegebenenfalls unter reduziertem Druck, durchgeführt.

In einer besonders bevorzugten Ausführungsform des erfindungsgemäßen Verfahrens wird die Herstellung der Organozinkhalogenide bei Refluxion zwischen 25 und 50 °C durchgeführt. Auf diese Weise können besonders hohe Produktausbeuten und Produktreinheiten erzielt werden.

Während der Zugabe des chiralen N-Auxiliars zu den Organozinkhalogeniden hält man die Temperatur der exothermen Reaktion gegebenenfalls durch Kühlung vorzugsweise auf einem vorgegebenen Wert. Die Zugabe erfolgt vorzugsweise bei Temperaturen von -80 bis +100 °C, besonders bevorzugt bei -50 bis +60 °C, insbesondere bei -30 bis +30 °C.

Zu den erhaltenen Mischungen aus Organozinkhalogeniden und dem chiralen N-Auxiliar wird dann ein Aldehyd oder Imin der allgemeinen Formel (2) zugegeben, gegebenenfalls gelöst in einem Lösungsmittel.

Alternativ können auch umgekehrt die erhaltenen Mischungen der Organozinkhalogenide und dem chiralen N-Auxiliar zu Aldehyden oder Iminen der allgemeinen Formel (2), gegebenenfalls gelöst in einem Lösungsmittel, zugegeben werden.

Es ist auch möglich, zu den Organozinkhalogeniden eine Mischung aus Aldehyd oder Imin der allgemeinen Formel (2) und des chiralen N-Auxiliars zuzugeben oder umgekehrt die Organozinkhalogenide zu einer Mischung der Aldehyde oder Imine der allgemeinen Formel (2) und dem chiralen N-Auxiliar zuzugeben.

Die Umsetzung der Organozinkhalogenide in Gegenwart des enatiomerenreinen, chiralen N-Auxiliars mit den Elektrophilen ausgewählt aus der Gruppe Aldehyde oder Imine erfolgt nach dem erfindungsgemäßen Verfahren bei einer Temperatur von unter 15 °C.

Es wurde überraschend gefunden, dass nur bei einer Temperatur von unter 15 °C hohe Produktausbeuten und gleichzeitig hohe chemische und optische Reinheiten erzielt werden können.

Guette et al. (M. Guette, J. Capillon, J.-P. Guette, Tetrahedron, 1973, 29, S. 3659) führen die Reaktion des chiralen Organozinkhalogenids - gebildet durch Umsetzung von Bromessigsäuremethyl- oder -ethylester mit Zink und Zugabe von (-)-Spartein - mit dem Elektrophil Benzaldehyd bei einer Temperatur von 20 °C durch, wobei zwar hohe optische Ausbeuten von 95±3 % ee aber nur sehr geringe chemische Ausbeuten von maximal 38+7 % erzielt wurden. Wird die Reaktion dagegen nach dem erfindungsgemäßen Verfahren bei einer Temperatur von kleiner 15 °C durchgeführt, dann wird überraschenderweise eine sehr hohe chemische Ausbeute von 97 % und gleichzeitig eine hohe optische Ausbeute von 89 % ee erzielt (vgl. Beispiel 6).

Dabei ist es unerheblich ob die Umsetzung durch Zugabe der Aldehyde oder Imine der allgemeinen Formel (2) zur Mischung des Organozinkhalogenids der allgemeinen Formel (3), wobei Hal hier Halogenozink bedeuten soll, und dem N- Auxiliar oder die Zugabe des Organozinkhalogenids zur Mischung von Aldehyden oder Iminen der allgemeinen Formel (2) und dem N-Auxiliar oder die Zugabe der Mischung von Organozinkhalogenid und N-Auxiliar zu den Aldehyden oder Iminen der allgemeinen Formel (2) erfolgt. Vorzugsweise erfolgt die Zugabe bei Temperaturen von -110 bis +10 °C, besonders bevorzugt bei -80 bis 0 °C, insbesondere bei -40 bis -10 °C.

Nach Ende der Zugabe aller beteiligten Bestandteile lässt man vorzugsweise noch 30 min bis 24 h, besonders bevorzugt 1 bis 18 h, insbesondere 2 bis 10 h nachreagieren, um die Umsetzung zu vervollständigen.

Überschüssiges Zinkmetall kann durch Filtration abgetrennt werden. Es ist auch möglich, überschüssiges Zink in der gegebenenfalls zur Hydrolyse der Reaktionsmischung verwendeten Säure aufzulösen.

Nach erfolgter Umsetzung wird die Reaktionsmischung üblicherweise bei Temperaturen von -110 bis +60 °C, besonders bevorzugt von -40 bis +10 °C gegebenenfalls nach Zugabe eines mit Wasser nicht mischbaren organischen Lösungsmittels, bevorzugt Toluol, Methylenchlorid, Ethylacetat oder tert.-Butylmethylether durch Zugabe von Wasser, einer wässrigen Säure oder Base hydrolysiert, wobei sich gebildete Zinkverbindungen und Zinksalze auflösen. Alternativ dazu kann man auch das Reaktionsgemisch zu Wasser, einer wässrigen Säure oder Base zusetzen.

Bevorzugte Basen sind Ammoniak und organische Amine, wie Trialkylamine und Alkanolamine.

Bevorzugte Säuren sind Brönstedt-Säuren, insbesondere starke Säuren, wie Bor-, Tetrafluorobor-, Salpetersäure, salpetrige Säure, Phosphorsäure, phosphorige Säure, unterphosphorige Säure, Schwefelsäure, schwefelige Säure, Peroxoschwefel-, Salz-, Fluss-, Jodwasserstoff-, Bromwasserstoff-, Perchlor-, Hexafluorophosphorsäure, Benzolsulfon-, p-Toluolsulfon-, Methansulfon-, Trifluormethansulfon- und Carbonsäuren, wie Chloressig-, Trichloressig-, Essig-, Acryl-, Benzoe-, Trifluoressig-, Citronen-, Croton-, Ameisen-, Fumar-, Malein-, Malon-, Gallus-, Itacon-, Milch-, Wein-, Oxal-, Phthal- und Bernsteinsäure.

Insbesondere wird Ammoniak, Salzsäure, Schwefelsäure, Essigsäure oder Citronensäure, bevorzugt Ammoniak, Salzsäure, Schwefelsäure und Essigsäure eingesetzt. Die Säure oder Base kann in konzentrierter Form oder in Form einer verdünnten wässrigen Lösung eingesetzt werden.

Alternativ zur Hydrolyse ist es möglich, die Reaktionsmischung üblicherweise bei Temperaturen von -110 bis +100 °C, bevorzugt bei -40 bis +50 °C mit einem Alkylierungs- oder Arylierungsreagens, einem Acylierungsreagens oder einem Silylierungsreagens, gegebenenfalls in Gegenwart eines Lösungsmittels, zu versetzen. Die so erhaltenen in 3-Oxy- oder 3-Amino-Position alkylierten, arylierten, silylierten oder acylierten Produkte der allgemeinen Formel (1a) oder (1b) können dann nach Hydrolyse oder gegebenenfalls direkt aus der Mischung z. B. durch Destillation, Extraktion oder Kristallisation einfach isoliert werden.

Als Alkylierungs- oder Arylierungs-, Acylierungs- oder Silylierungsreagenzien finden alle hierfür geeigneten, dem Fachmann aus dem Stand der Technik wohlbekannten Reagenzien Verwendung.

Bevorzugte Alkylierungsmittel sind Methyl-, Ethyl-, Propyl-, Butyl-, Benzylchlorid, -bromid oder -iodid.

Bevorzugte Arylierungsreagenzien sind Fluorbenzol, 1-Fluor-4-trifluormethylbenzol, 1-Fluornaphthalin, Chlorbenzol, 1-Chlor-4-trifluormethylbenzol, oder 1-Chlornaphthalin, insbesondere 1-Fluornaphtalin oder 1-Fluor-4-trifluormethylbenzol.

Bevorzugte Acylierungsreagenzien sind Acetylchlorid, -bromid und Acetanhydrid, insbesondere Acetanhydrid.

Bevorzugte Silylierungsreagenzien sind Trimethyl-, -ethyl-, propyl-, -butylchlorsilan und tert.-Butyldimethylchlorsilan, insbesondere Trimethylsilan.

Werden als reaktive Halogenverbindungen der allgemeinen Formel (3) Silylverbindungen mit **Y** = **(C=O)-Z** und **Z** = **OSi(R**^{**1**}**)**_{**3**} eingesetzt, wobei **R**^{**1**} die oben genannten Bedeutungen haben kann, insbesondere aus den oben genannten bevorzugten Ausführungsformen für R¹ ausgewählt wird, dann werden durch anschließende Hydrolyse unter den oben genannten Bedingungen Carbonsäuren erhalten, wobei **Z** hier **OH** bedeutet.

Die so erhaltenen Produkte der allgemeinen Formel (1a) oder (1b) können zuletzt aus der organischen Phase der Reaktionsmischung nach Phasentrennung isoliert werden. Aus der organischen Phase können diese nach bekannten, üblicherweise verwendeten Methoden wie Extraktion, Destillation, Kristallisation oder mittels chromatographischer Methoden isoliert werden. In den meisten Fällen ist bereits das nach Entfernung des Lösungsmittels erhaltene Rohprodukt von sehr hoher Reinheit.

Gegebenenfalls werden kristalline Verbindungen der allgemeinen Formeln (1a) oder (1b) zur gegebenenfalls weiteren Enantiomerenanreicherung umkristallisiert. Zur Umkristallisation werden die Reaktionsprodukte bevorzugt in Form ihrer Rohprodukte in einem geeigneten Lösungsmittel gelöst und gegebenenfalls durch Animpfen mit enantiomerenreiner Verbindung umkristallisiert.

Wie eingangs beschrieben wurde gefunden, dass sich das chirale N-Auxiliar bei der Aufarbeitung der Reaktionsmischung als zinkhaltiger Feststoff isolieren und daraus in hohen Ausbeuten wiedergewinnen lässt. Die Aufarbeitung kann dabei bevorzugt wässrig, sauer, alkalisch oder mit einem Silylierungs-, Alkylierungs-, Arylierungs- oder Acylierungsregenz erfolgen, insbesondere unter nicht-wässrigen Bedingungen mit einem Silylierungsreagenz.

Durch die Abtrennung des zinkhaltigen Feststoffes von der Reaktionsmischung lässt sich einerseits der größte Teil des Zinks aus dem Reaktionsgemisch entfernen, so dass keine zinkhaltigen Lösungen oder sonstige zinkhaltigen Abfälle anfallen und andererseits lässt sich aus dem abgetrennte zinkhaltigen Feststoff das eingesetzte N-Auxiliar in hohen Ausbeuten wiedergewinnen und regenerieren.

Insbesondere lässt sich die Wiedergewinnung bei der Verwendung von (-)-Spartein als N-Auxiliars mit hohen Ausbeuten durchführen, insbesondere bei der Aufarbeitung mit einem Silylierungsreagenz.

Die einfache und effektive Rückgewinnung des chiralen N-Auxiliars, insbesondere von (-)-Spartein, macht das erfindungsgemäße Verfahren besonders wirtschaftlich.

Das N-Auxiliar, insbesondere (-)-Spartein, kann vor Wiedereinsatz destillativ von Verunreinigungen befreit und gereinigt werden. Besonders bevorzugt wird (-)-Spartein ohne destillative Reinigung wieder eingesetzt.

Es hat sich bewährt, das Zink mit der reaktiven Halogenverbindung der allgemeinen Formel (3), dem Aldehyd oder Imin und dem chiralen N-Auxiliar im Molverhältnis (1 bis 3) : (1 bis 2) : 1 : (0.1 bis 2.5), insbesondere (1.1 bis 1.7) : (1 bis 1.3) : 1 : (0.1 bis 1.5) umzusetzen.

Zink wird im allgemeinen in Form von Folien, Band-, Span-, Pulver- oder Staubform oder in Form von Zinkwolle eingesetzt. Die Anwesenheit andere Metalle wie Kupfer, Silber oder Quecksilber ist nicht erforderlich. Insbesondere wird Zink in Form von kommerziell erhältlichem, handelsüblichem Zinkpulver oder Zinkstaub eingesetzt, bevorzugt ist Zink hoher Reinheit von mindestens 99.995 %,besonders bevorzugt ist Zinkstaub, der aus Zink mit einer Reinheit von mindestens 99.995 % gewonnen wird.

Zur Erzielung hoher Produktausbeuten und -reinheiten hat es sich bewährt, das Zink vor Zugabe der reaktiven Halogenverbindung der allgemeinen Formel (3) oder des chiralen N-Auxiliars bzw. deren Mischung zu aktivieren. Zur Zinkaktivierung sind vorbekannte, beispielsweise in der zusammenfassenden Darstellung von A. Fürstner, Synthesis 1989, S. 571, genannte, üblicherweise verwendete Methoden geeignet. Bewährt haben sich die Wäsche des Zinks mit Säure, die Aktivierung durch Iod wie in EP-A-562 343 beschrieben und die Aktivierung durch Trimethylchlorsilan, wobei die Aktivierung durch Trimethylchlorsilan aufgrund ihrer einfachen Durchführbarkeit und der gesteigerten Ausbeuten, Produktreinheiten und Selektivitäten sowie der Unterdrückung von Nebenreaktionen besonders bevorzugt ist. Aus G. Picotin, P. Miginiac, J. Org. Chem. 1987, 52, S. 4796, ist die Aktivierung von Zink durch Trimethylchlorsilan in dem Lösungsmittel Diethylether bekannt.

Zur Aktivierung von Zink mit Trimethylchlorsilan wird das Zink in dem Lösungsmittel oder Lösungsmittelgemisch vorgelegt, dann Trimethylchlorsilan zugegeben und die Mischung für 10 min bis 2 h, bevorzugt 5 bis 45 min bei Temperaturen von 20 bis 150 °C, insbesondere bei 30 bis 120 °C, bevorzugt bei 40 bis 80 °C erhitzt. Es hat sich bewährt, das Zink mit Trimethylchlorsilan im Molverhältnis 1 : (0.01 bis 0.5), insbesondere 1 : (0.05 bis 0.3) unter Erwärmung auf die gewünschte Temperatur umzusetzen.

Zur Aktivierung der Reaktionsmischung können auch Zusatzstoffe wie Kupfer-, Chrom-, Mangan-, Cobalt-, Bismut-, Samarium-, Scandium-, Indium-, Titan-, Cer-, Tellur-, Zinn-, Blei-, Antimon-, Germanium-, Aluminium-, Magnesium-, Palladium-, Nickel- und Quecksilberverbindungen oder gegebenenfalls deren Mischungen eingesetzt werden.

Der Druckbereich der Reaktion ist unkritisch und kann innerhalb weiter Grenzen variiert werden. Der Druck beträgt üblicherweise 0.01 bis 20 bar, bevorzugt wird die Reaktion unter Normaldruck (Atmosphärendruck) durchgeführt.

Die Reaktion wird bevorzugt unter Inertisierung mit Schutzgas, wie Stickstoff oder Argon durchgeführt. Die Reaktion kann kontinuierlich oder diskontinuierlich, bevorzugt diskontinuierlich durchgeführt werden.

Alle vorstehenden Symbole der vorstehenden Formeln weisen ihre Bedeutungen jeweils unabhängig voneinander auf.

In den folgenden Beispielen sind, falls jeweils nicht anders angegeben, alle Mengen- und Prozentangaben auf das Gewicht bezogen, alle Drücke 0,10 MPa (abs.) und alle Temperaturen 20°C.

### Beispiel 1:

### Herstellung von (S)-(-)-3-Hydroxy-3-(2-thiophen)propionsäuremethylester

Bei Raumtemperatur wurden in einem Dreihalskolben mit Rückflusskühler, Innenthermometer, Tropftrichter und Rührer unter Stickstoff-Schutzgas 9 g Zinkstaub (138 mmol) in 70 ml Tetrahydrofuran vorgelegt. Nachdem 2.3 ml Trimethylchlorsilan zugefügt wurden, erwärmte man die Mischung 15 min auf 60 °C und tropfte anschließend innerhalb 7 min bei 45 °C 20.4 g Bromessigsäuremethylester (134 mmol) unverdünnt zu. Dann wurde die Mischung 15 min gerührt und mit 20 ml 1-Methyl-2-pyrrolidon versetzt. Nach Abkühlung auf 0 °C wurden 32.6 g (-)-Spartein (139 mmol) unverdünnt zugegeben, wobei die Temperatur auf 8 °C-anstieg. Nachdem die klare Mischung 10 min bei 40 °C gerührt wurde, kühlte man auf -20 °C ab und fügte 12.4 g Thiophen-2-carbaldehyd (111 mmol) unverdünnt zu. Die klare Reaktionsmischung wurde anschließend 8 h bei -20 °C gerührt, dann bei dieser Temperatur mit 35 ml 20 %-iger Salzsäure bis zu einem pH-Wert von 2 angesäuert und 10 min gerührt, wobei die Temperatur der Mischung auf 0 °C anstieg. Dann wurden 150 ml Wasser zugefügt. Ausgefallenes Spartein-Salz wurde abgesaugt und zweimal mit je 40 ml Ethylacetat gewaschen (Spartein konnte zu > 80 % zurückgewonnen werden). Nach Abtrennung der organischen Phase extrahierte man die wässrige Phase mit 50 ml Ethylacetat und trennte anschließend die organische Phase ab. Die organische Phase wurde dann bei -10 °C 10 min mit 20 ml konzentrierter Ammoniak-Lösung gerührt. Nach Phasentrennung wurde das Lösungsmittel im Vakuum weitestgehend abdestilliert und der Rückstand mit 20 ml Wasser gewaschen. Nach Phasentrennung, Trocknung und Entfernung von Lösungsmittel im Vakuum wurde (S)-(-)-3-Hydroxy-3-(2-thiophen)propionsäuremethylester in einer Ausbeute von 19.8 g (96 % d. Th.) und einer Reinheit von > 94 % und 91 % ee (HPLC) und einem Siedepunkt von 89 °C (0.6 mbar) erhalten.

### Beispiel 2:

### Herstellung von (S)- (-)-3-Hydroxy-3-(2-thiophen)propionsäureethylester

Bei Raumtemperatur wurden in einem Dreihalskolben mit Rückflusskühler, Innenthermometer, Tropftrichter und Rührer unter Stickstoff-Schutzgas 9 g Zinkstaub (138 mmol) in 60 ml Tetrahydrofuran vorgelegt. Nachdem 2.3 ml Trimethylchlorsilan zugefügt wurden, erwärmte man die Mischung 15 min auf 60 °C und tropfte anschließend innerhalb 7 min bei 45 °C 22.4 g Bromessigsäureethylester (134 mmol) unverdünnt zu. Dann wurde die Mischung 15 min gerührt. Nach Abkühlung auf 0 °C wurden 60 ml Dimethylformamid zugefügt und anschließend 32.6 g (-)-Spartein (139 mmol) unverdünnt zugegeben, wobei die Temperatur auf 8 °C anstieg. Nachdem die klare Mischung 10 min bei 10 °C gerührt wurde, kühlte man auf -20 °C ab und fügte 12.4 g Thiophen-2-carbaldehyd (111 mmol) unverdünnt zu. Die klare Reaktionsmischung wurde anschließend 8 h bei -20 °C gerührt, dann bei dieser Temperatur mit 35 ml 20 %-iger Salzsäure bis zu einem pH-Wert von 2 angesäuert und 10 min gerührt, wobei die Temperatur der Mischung auf 0 °C anstieg. Dann wurden 150 ml Wasser zugefügt. Ausgefallenes Spartein-Salz wurde abgesaugt und zweimal mit je 40 ml Ethylacetat gewaschen (Spartein konnte zu > 80 % zurückgewonnen werden). Nach Abtrennung der organischen Phase extrahierte man die wässrige Phase mit 50 ml Ethylacetat und trennte anschließend die organische Phase ab. Die organische Phase wurde dann bei -10 °C 10 min mit 20 ml konzentrierter Ammoniak-Lösung gerührt. Nach Phasentrennung, Trocknung und Entfernung von Lösungsmittel im Vakuum wurde (S)-(-)-3-Hydroxy-3-(2-thiophen)propionsäureethylester in einer Ausbeute von 20.9 g (94 % d. Th.) und einer Reinheit von > 94 % und 89 % ee (HPLC) erhalten.

### Beispiel 3:

### Herstellung von (S)-(-)-3-(2-Thiophen)-3-trimethylsiloxypropionsäuremethylester

Bei Raumtemperatur wurden in einem Dreihalskolben mit Rückflusskühler, Innenthermometer, Tropftrichter und Rührer unter Stickstoff-Schutzgas 5 g Zinkstaub (77 mmol) in 30 ml Tetrahydrofuran vorgelegt. Nachdem 1.2 ml Trimethylchlorsilan zugefügt wurden, erwärmte man die Mischung 15 min auf 55 °C und tropfte anschließend innerhalb 7 min bei 40 °C unter Rückfluss 10.9 g Bromessigsäuremethylester (71 mmol) unverdünnt zu. Dann wurde die Mischung 10 min gerührt. Nach Abkühlung auf 20 °C wurden 30 ml Tetrahydrofuran zugefügt und anschließend bei 0 °C 17 g (-)-Spartein (72 mmol) unverdünnt zugegeben, wobei die Temperatur auf 10 °C anstieg. Man kühlte auf -15 °C ab und fügte 6.6 g Thiophen-2-carbaldehyd (60 mmol) unverdünnt zu. Die Reaktionsmischung wurde anschließend 20 h bei -15 °C gerührt, dann bei dieser Temperatur mit 8 g Trimethylchlorsilan (74 mmol) versetzt, wobei die Temperatur der Mischung auf 10 °C anstieg und sich ein Niederschlag bildete. Die Mischung wurde anschließend 1 h bei 20 °C gerührt, organisches Lösungsmittel im Vakuum zur Hälfte abdestilliert, der Rückstand mit 70 ml Pentan versetzt, 30 min gerührt und auf 0 °C abgekühlt. Ausgefallenes Spartein-Salz wurde abgesaugt und zweimal mit je 50 ml Pentan gewaschen (Spartein konnte zu > 90 % zurückgewonnen werden). Die organische Produktphase wurde mit 5 ml gesättigter Natriumhydrogencarbonat-Lsg. gewaschen. Nach Phasentrennung, Trocknung und Entfernung von Lösungsmittel im Vakuum wurde (S)-(-)-3-(2-Thiophen)-3-trimethylsiloxypropionsäuremethylester in einer Ausbeute von 15 g (98 % d. Th.)und einer Reinheit von > 95 % und 89 % ee (HPLC) erhalten. Drehwert: α_{D} = -54.3 (20 °C, Methylenchlorid, c = 4); Siedepunkt: 70-71 °C (0.04 mbar).

### Beispiel 4:

### Herstellung von (S)-(-)-3-Methoxy-3-(2-thiophen)propionsäuremethylester

Bei Raumtemperatur wurden in einem Dreihalskolben mit Rückflusskühler, Innenthermometer, Tropftrichter und Rührer unter Stickstoff-Schutzgas 5 g Zinkstaub (77 mmol) in 30 ml Tetrahydrofuran vorgelegt. Nachdem 1.2 ml Trimethylchlorsilan zugefügt wurden, erwärmte man die Mischung 15 min auf 55 °C und tropfte anschließend innerhalb 7 min bei 45 °C 10.9 g Bromessigsäuremethylester (71 mmol) unverdünnt zu. Dann wurde die Mischung 10 min gerührt. Nach Abkühlung auf 20 °C wurden 30 ml Tetrahydrofuran zugefügt und anschließend bei 0 °C 17 g (-)-Spartein (72 mmol) unverdünnt zugegeben, wobei die Temperatur auf 10 °C anstieg. Man kühlte auf -15 °C ab und fügte 6.6 g Thiophen-2-carbaldehyd (60 mmol) unverdünnt zu. Die Reaktionsmischung wurde anschließend 20 h bei -15 °C gerührt, dann bei dieser Temperatur mit 10.5 g Methyljodid (74 mmol) versetzt, wobei die Temperatur der Mischung auf 0 °C anstieg. Die Mischung wurde anschließend 1 h bei 20 °C gerührt, organisches Lösungsmittel im Vakuum zur Hälfte abdestilliert und auf 0 °C abgekühlt. Ausgefallenes Spartein-Salz wurde abgesaugt und zweimal mit je 50 ml Pentan gewaschen (Spartein konnte zu > 90 % zurückgewonnen werden). Die organische Produktphase wurde mit 5 ml gesättigter Natriumhydrogencarbonat-Lsg. gewaschen. Nach Phasentrennung, Trocknung und Entfernung von Lösungsmittel im Vakuum wurde (S)-(-)-3-Methoxy-3-(2-thiophen)propionsäuremethylester in einer Ausbeute von 11.1 g (92 % d. Th.) und 89 % ee (HPLC) erhalten.

### Beispiel 5:

### Herstellung von (S)-(-)-3-Acetoxy-3-(2-thiophen)propionsäuremethylester

Bei Raumtemperatur wurden in einem Dreihalskolben mit Rückflusskühler, Innenthermometer, Tropftrichter und Rührer unter Stickstoff-Schutzgas 5 g Zinkstaub (77 mmol) in 30 ml Tetrahydrofuran vorgelegt. Nachdem 1.2 ml Trimethylchlorsilan zugefügt wurden, erwärmte man die Mischung 15 min auf 55 °C und tropfte anschließend innerhalb 7 min bei 45 °C 10.9 g Bromessigsäuremethylester (71 mmol) unverdünnt zu. Dann wurde die Mischung 10 min gerührt. Nach Abkühlung auf 20 °C wurden 30 ml Tetrahydrofuran zugefügt und anschließend bei 0 °C 17 g (-)-Spartein (72 mmol) unverdünnt zugegeben, wobei die Temperatur auf 10 °C anstieg. Man kühlte auf -15 °C ab und fügte 6.6 g Thiophen-2-carbaldehyd (60 mmol) unverdünnt zu. Die Reaktionsmischung wurde anschließend 20 h bei -15 °C gerührt, dann bei dieser Temperatur mit 5.8 g Acetylchlorid (74 mmol) versetzt, wobei die Temperatur der Mischung auf 10 °C anstieg. Die Mischung wurde anschließend 1 h bei 20 °C gerührt, organisches Lösungsmittel im Vakuum zur Hälfte abdestilliert, der Rückstand mit 70 ml Pentan versetzt, 30 min gerührt und auf 0 °C abgekühlt. Ausgefallenes Spartein-Salz wurde abgesaugt und zweimal mit je 50 ml Pentan gewaschen (Spartein konnte zu > 90 % zurückgewonnen werden). Die organische Produktphase wurde mit 5 ml gesättigter Natriumhydrogencarbonat-Lsg. gewaschen. Nach Phasentrennung, Trocknung und Entfernung von Lösungsmittel im Vakuum wurde (S)-(-)-3-Acetoxy-3-(2-thiophen)propionsäuremethylester in einer Ausbeute von 13.1 g (93 % d. Th.) und 89 % ee (HPLC) erhalten.

### Beispiel 6:

### Herstellung von (S)-(-)-3-Hydroxy-3-phenylpropionsäuremethylester

Bei Raumtemperatur wurden in einem Dreihalskolben mit Rückflusskühler, Innenthermometer, Tropftrichter und Rührer unter Stickstoff-Schutzgas 5 g Zinkstaub (77 mmol) in 30 ml Tetrahydrofuran vorgelegt. Nachdem 1.2 ml Trimethylchlorsilan zugefügt wurden, erwärmte man die Mischung 15 min auf 55 °C und tropfte anschließend innerhalb 7 min bei 40 °C unter Rückfluss 10.9 g Bromessigsäuremethylester (71 mmol) unverdünnt zu. Dann wurde die Mischung 10 min gerührt. Nach Abkühlung auf 10 °C wurden 30 ml Tetrahydrofuran zugefügt und anschließend bei 0 °C 17 g (-)-Spartein (72 mmol) unverdünnt zugegeben, wobei die Temperatur auf 5 °C anstieg. Man kühlte auf -15 °C ab und fügte 6.4 g Benzaldehyd (60 mmol) unverdünnt zu. Die Reaktionsmischung wurde anschließend 20 h bei -15 °C gerührt, dann bei dieser Temperatur mit 35 ml 20 %-iger Salzsäure bis zu einem pH-Wert von 3 angesäuert und 10 min gerührt, wobei die Temperatur der Mischung auf 0 °C anstieg. Dann wurden 150 ml Wasser zugefügt. Ausgefallenes Spartein-Salz wurde abgesaugt und zweimal mit je 40 ml Ethylacetat gewaschen. Nach Abtrennung der organischen Phase extrahierte man die wässrige Phase mit 50 ml Ethylacetat und trennte anschließend die organische Phase ab. Die organische Phase wurde dann bei -10 °C 5 min mit 20 ml konzentrierter Ammoniak-Lösung gerührt. Nach Phasentrennung, Trocknung und Entfernung von Lösungsmittel im Vakuum wurde (S)-(-)-3-Hydroxy-3-phenylpropionsäuremethylester in einer Ausbeute von 10.5 g (97 % d. Th.) und einer Reinheit von > 95 % und 89 % ee (HPLC) erhalten.

In analoger Weise wurden folgende Verbindungen hergestellt:

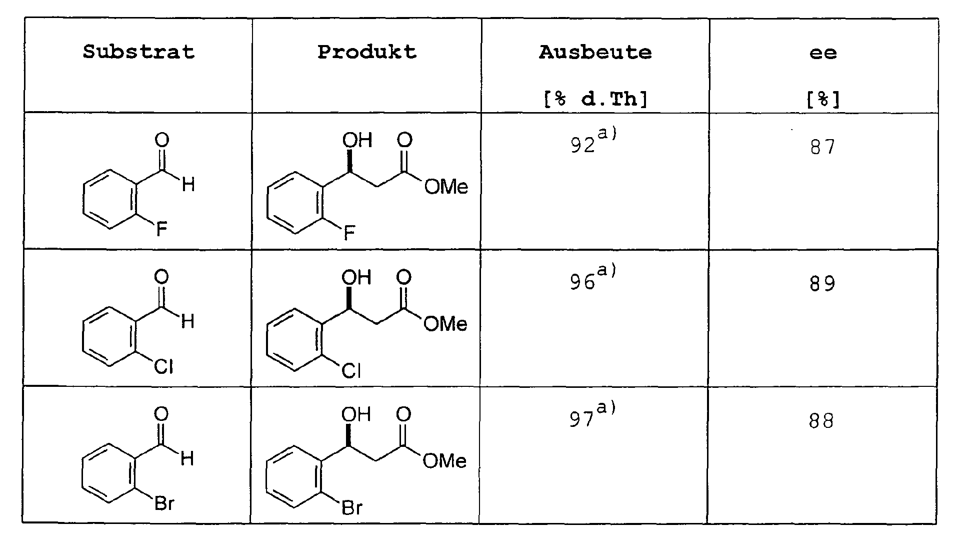

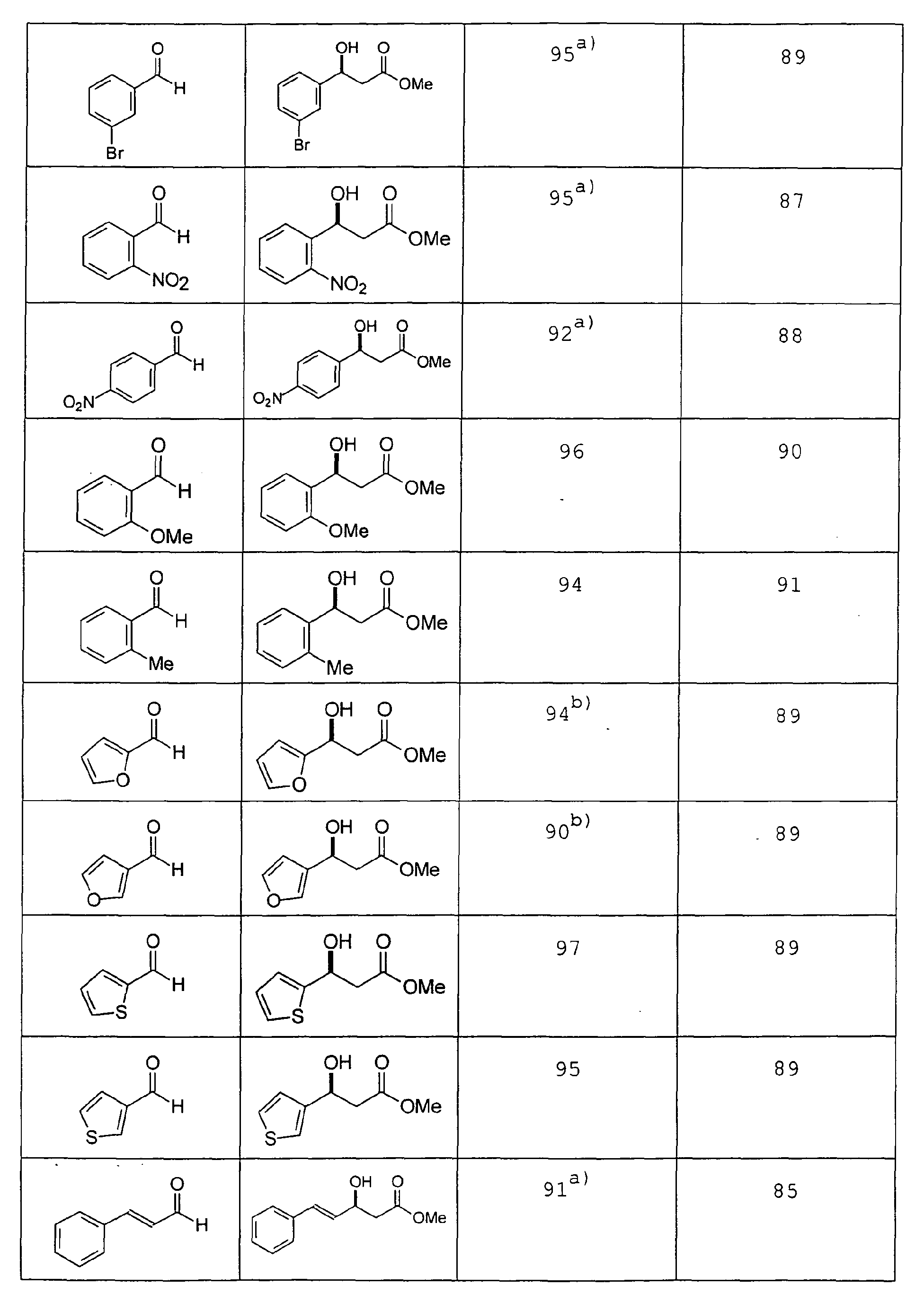

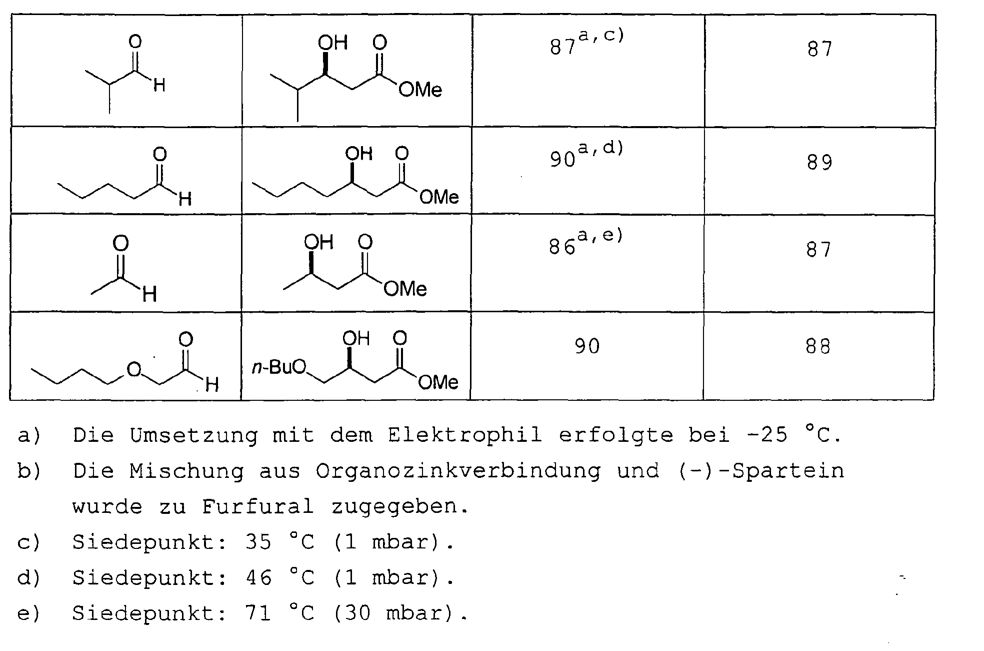

## Patentansprüche

1. Verfahren zur Herstellung von Verbindungen der allgemeinen Formel (1a) oder (1b) 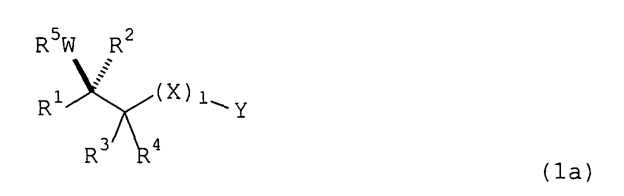 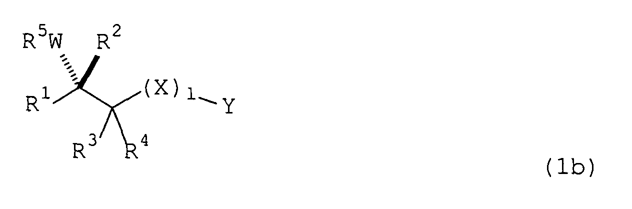 wobei
**R**^{**1**} und **R**^{**2**} unabhängig voneinander Wasserstoff oder einen gegebenenfalls halogen- oder cyanosubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S- oder -N**R**^{**x**}- ersetzt sein können und in dem eine oder mehrere Methineinheiten durch Gruppen -N= oder -P= ersetzt sein können,
**R**^{**3**} und **R**^{**4**} unabhängig voneinander Wasserstoff,.Halogen oder einen gegebenenfalls halogensubstituierten oder cyanosubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S- oder -N**R**^{**x**}- ersetzt sein können und in dem eine oder mehrere Methineinheiten durch Gruppen -N= oder -P= ersetzt sein können, **R**^{**x**} Wasserstoff oder einen gegebenenfalls halogensubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S-, -NHoder -N-C₁-C₂₀-Alkyl ersetzt sein können und in dem eine oder mehrere Methineinheiten durch Gruppen -N= oder -P= ersetzt sein können, bedeuten und
**X** ausgewählt wird aus 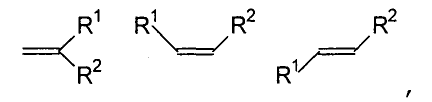
**l** für eine ganze Zahl im Wert von 0 oder 1 steht,
**W** O oder NR⁶,
**R**^{**5**} Wasserstoff oder eine Alkyl-, Aryl-, Acyl- oder Silylgruppe und
**R**^{**6**} Wasserstoff, Si(R¹)₃ oder einen gegebenenfalls halogenoder cyanosubstituierten C₁-C₃₀-Kohlenwasserstoffrest bedeuten kann, in dem eine oder mehrere, einander nicht benachbarte Methyleneinheiten durch Gruppen -O-, -CO-, -COO-, -OCO-, oder -OCOO-, -S-, -SO-, -SO₂-, -P(R¹)-, -P(OR¹)-,-PO(R¹)-, -PO(OR¹)-, oder -NR^{x}- ersetzt sein können und in dem eine oder mehrere Methineinheiten durch Gruppen -N= oder -P= ersetzt sein können,
**Y** für CN, (C=O)-Z, (SO₂)-Z, (P=O)(-Z)₂ oder einen aromatischen Rest steht, wobei eine oder mehrere Methineinheiten im Ring durch Gruppen -N= oder -P= ersetzt sein können und der Ring die Heteroatome -O-, -S- oder -NH- tragen kann, wobei der aromatische Ring gegebenenfalls halogen- oder cyanosubstituiert oder durch C₁-C₃₀-Kohlenwasserstoffreste, in denen eine oder mehrere, einander nicht benachbarte Methyleneinheiten durch Gruppen -O-, -CO-, -COO-, -OCO-, oder -OCOO-, -S- oder -NR^{x}- ersetzt sein können, substituiert ist und **Z** für einen gegebenenfalls halogensubstituierten C₁-C₃₀-Kohlenwasserstoffrest steht, in dem eine oder mehrere, einander nicht benachbarte Methyleneinheiten durch Gruppen -O-, -CO-, -COO-, -OCO-, oder -OCOO-, -S-, oder -NR^{x}- ersetzt sein können und in dem eine oder mehrere Methineinheiten durch Gruppen -N= oder -P= ersetzt sein können, oder für OH, OR¹, OSi(R¹)₃, NHR¹ oder NR¹R² steht
**dadurch gekennzeichnet, dass**
Aldehyde oder Imine der allgemeinen Formel (2)
R¹R²C=W (2)
bei einer Temperatur von kleiner 15 °C
in Gegenwart eines chiralen, enantiomerenreinen Auxiliars mit mindestens zwei Stickstoff-Donatoren und mindestens einer cyclischen Gruppe mit der Maßgabe, dass vorhandene Protonen einen pKa größer 18 haben
mit einem Organozinkhalogenid umgesetzt wird, welches aus
Zink und
einer reaktiven Halogenverbindung der allgemeinen Formel (3)
Hal-R³R⁴C-(X)₁-Y (3)
wobei
**Hal** Chlor, Brom oder Iod bedeutet, erzeugt wird,
und anschließend Wasser, Säure oder Base oder ein Alkylierungs-, Arylierungs-, Acylierungs- oder Silylierungsreagens zugegeben wird.

2. Verfahren nach Anspruch 1 **dadurch gekennzeichnet, dass** die Reaktion in einem Lösungsmittel ausgewählt aus der Gruppe der cyclische Ether, Di- oder Oligoether mit mindestens einer Ethylen-Brücke, Carbonsäureester, Amine, Säureamide, polar aprotischen Lösungsmittel oder halogenierten Kohlenwasserstoffe oder Lösungsmittelgemischen enthaltend eines oder mehrere dieser Lösungsmittel ausgewählt aus der Gruppe durchgeführt wird.

3. Verfahren nach Anspruch 1 bis 2 **dadurch gekennzeichnet, dass** das chirale, enantiomerenreine Auxiliar (-)-Spartein ist.

4. Verfahren nach Anspruch 1 bis 3 **dadurch gekennzeichnet, dass** die Herstellung des für die Umsetzung benötigten Reformatsky-Reagenzes durch die Vorlage von Zink in einem Lösungsmittel oder Lösungsmittelgemisch, die anschließende Zugabe der reaktiven Halogenverbindung, gegebenenfalls gelöst in einem Lösungsmittel und die abschließende Zugabe des chiralen, enantiomerenreinen Auxiliars, gegebenenfalls gelöst in einem Lösungsmittel, erfolgt.

5. Verfahren nach Anspruch 4 **dadurch gekennzeichnet, dass** die Herstellung der Organozinkhalogenide bei 25 bis 50 °C erfolgt.

6. Verfahren nach einem der Ansprüche 4 oder 5, **dadurch gekennzeichnet, dass** die Zugabe des chiralen, enatiomerenreinen Auxiliars zu den Organozinkhalogeniden bei -30°C bis +30°C erfolgt.

7. Verfahren nach Anspruch 1 bis 6, **dadurch gekennzeichnet, dass** die Umsetzung der Aldehyde oder Imine mit dem Organozinkhalogenid in Gegenwart des chiralen, entiomerenreinen Auxiliars bei einer Temperatur von -10 °C bis -40°C erfolgt.

8. Verfahren nach Anspruch 1 bis 7, **dadurch gekennzeichnet, dass** das Zink aus der Reaktionslösung in Form eines Zink und das chirale, enantiomerenreine Auxiliar enthaltenden Feststoffes abgetrennt wird.

9. Verfahren nach Anspruch 8, **dadurch gekennzeichnet, dass** das chirale, enantiomerenreine Auxiliar aus dem Feststoff wiedergewonnen wird.

10. Verfahren nach Anspruch 1 bis 9, **dadurch gekennzeichnet, dass** als reaktive Halogenverbindungen α-Bromessigsäureester verwendet werden.
